# EUROPEAN PATENT SPECIFICATION

(11) **EP 3 127 902 B1**
(45) Date of publication and mention of the grant of the patent: **07.11.2018**
(21) Application number: 15772182.0
(22) Date of filing: 30.03.2015
(51) Int. Cl.: C07D 275/02

(54) **METHOD FOR PRODUCING ISOTHIAZOLE COMPOUND USING ETHER COMPOUND**
VERFAHREN ZUR HERSTELLUNG EINER ISOTHIAZOLVERBINDUNG MIT EINER ETHERVERBINDUNG
PROCÉDÉ DE PRODUCTION D'UN COMPOSÉ ISOTHIAZOLE À L'AIDE D'UN COMPOSÉ ÉTHER

(30) Priority: 03.04.2014 JP 2014076906
(43) Date of publication of application: 08.02.2017
(73) Proprietor: Kumiai Chemical Industry Co., Ltd., Tokyo (JP)
(72) Inventor: ABE, Takashi, 2256, Nakanogo, Fuji-shi Shizuoka 421-3306 (JP)
(74) Representative: Laufhütte, Dieter
(86) International application number: PCT/JP2015/001815
(87) International publication number: WO 2015/151491

(56) References cited:
- WO-A1-2010/126170
- WO-A1-2014/054294
- DE-A1- 2 231 097
- DE-A1- 2 231 098
- JP-A- 2010 260 805
- US-A- 3 341 547

## Description

### Technical Field

The present invention relates to a process for producing an isothiazole compound. Isothiazole compounds are useful, for example, as intermediates for the synthesis of various organic compounds (e.g., biologically active organic compounds such as pharmaceuticals and agricultural chemicals, functional pigments, electronic materials, etc.) because of their structure of isothiazole.

### Background Art

As described above, isothiazole compounds are widely known as intermediates for pharmaceuticals and intermediates for agricultural chemicals, and intermediates for functional pigments, electronic materials, etc. Therefore, as disclosed in Non-Patent Documents 1 and 2, various studies have heretofore been made on processes for producing isothiazole compounds.

Among isothiazole compounds, 3,4-dichloro-5-cyanoisothiazole, which can easily be subjected to the conversion of a functional group thereof, is known as an intermediate for pharmaceuticals and an intermediate for agricultural chemicals. In addition, as disclosed in Patent Documents 4 and 5, this compound is in fact used as an important intermediate for agricultural chemicals.

However, it has been difficult for the production processes disclosed in Non-Patent Documents 1 and 2 to produce 3,4-dichloro-5-cyanoisothiazole, which is useful as an important intermediate for agricultural chemicals.

That is, a process using carbon disulfide (CS₂), sodium cyanide (NaCN) and chlorine (Cl₂) has heretofore been known as a process for producing 3,4-dichloro-5-cyanoisothiazole (see Patent Document 1). However, this process has a drawback that carbon disulfide, which is a special inflammable material, is used as a raw material to be used therein. Moreover, this process also has a drawback that sodium cyanide, which is a toxic material, is used therein. Furthermore, in this process, chlorine is introduced into a reactor containing therein N,N-dimethylformamide (DMF) as a solvent with heating. However, it is well known to a person skilled in the art that when N,N-dimethylformamide and chlorine are used simultaneously, there is a possibility of the runaway of the reaction or an explosion. For these reasons, it is considered that the implementation of this process requires most careful attention and adequate measures in order to maintain safety. In addition, there is a possibility that this process cannot ensure the safety of a production plant because there is a possibility that the runaway of the reaction and an explosion occur in some cases as described above. That is, this process using N,N-dimethylformamide and chlorine at the same time is not preferred for industrial manufacture because there is concern for lack of safety.

As another process for producing 3,4-dichloro-5-cyanoisothiazole, a process using trichloroacetonitrile and sulfur is known (see Patent Document 2). However, this process has the drawback of requiring the reaction at a high temperature of 200 to 300°C as described in Examples therein. In addition, this process has the drawback of requiring the use of a special raw material such as trichloroacetonitrile.

Furthermore, a process using dichlorofumaronitrile and sulfur is known (see Patent Document 3). However, this process also has the drawback of requiring the reaction at a high temperature of 230 to 300°C in Examples therein. In addition, this process also has the drawback of requiring the use of a special raw material such as dichlorofumaronitrile.

As still another production process, a process of reacting fumaronitrile, maleonitrile or a chlorine-substituted compound thereof, or a mixture of these compounds with sulfur chloride in an aprotic polar solvent is known (see Patent Document 6). Fumaronitrile, maleonitrile and a chlorine-substituted compound thereof, or a mixture of these compounds, which is/are used in this process, can be produced from succinonitrile (see Examples 7 and 8 of Patent Document 6). However, it is desired that the production process described in Patent Document 6 is further improved in that this process requires two steps from succinonitrile.

In addition, it is considered that fumaronitrile, maleonitrile or a chlorine-substituted compound thereof has an industrially significant sublimation property. Compounds having a sublimation property generally have the potential of causing clogging of a reflux condenser or a pipeline in a plant by sublimation thereof. For this reason, the process described in Patent Document 6 has the drawback of having the possibility of requiring attention and measures in operations in its industrial implementation.

Besides, this process essentially requires an aprotic polar solvent such as N,N-dimethylformamide. And the recycle of the aprotic polar solvent is accompanied by difficulty because of working-up using water. Therefore, there is a drawback that it is highly possible that the used aprotic polar solvent becomes a part of waste. In addition, in this process, there is an example in which N,N-dimethylformamide and sulfur chloride, which is a chlorine compound, are used simultaneously. Therefore, there is a possibility that this process requires attention and measures with the view of preparing for any situation. Therefore, there is still room for improvement in this process.

Meanwhile, as a production process of 3,4-dichloro-5-cyanoisothiazole, a process using succinonitrile, sulfur and chlorine is known (see Patent Document 7). However, this process also requires N,N-dimethylformamide as a solvent. That is, since N,N-dimethylformamide and chlorine are used simultaneously, there is a possibility of the runaway of the reaction and an explosion. For this reason, it is considered that the implementation of this process also requires most careful attention and adequate measures in order to maintain safety. In addition, there is a possibility that this process cannot ensure the safety of a production plant because there is a possibility that the runaway of the reaction and an explosion occur in some cases as described above. That is, this process using N,N-dimethylformamide and chlorine at the same time is not preferred for industrial manufacture because there is concern for lack of safety.

Besides, the process described in Patent Document 7 essentially requires an aprotic polar solvent such as N,N-dimethylformamide. And the recycle of the aprotic polar solvent is accompanied by difficulty because of working-up using water. Therefore, there is a drawback that it is highly possible that the used aprotic polar solvent becomes a part of waste. That is to say, there is still room for improvement in this process.

### Citation List

### Patent Document

Patent Document 1: US 3341547 A
Patent Document 2: DE 2231097 A (DT 2231097)
Patent Document 3: DE 2231098 A (DT 2231098)
Patent Document 4: Japanese Patent Application Laid-Open No. Hei-5-59024 (JP-A-1993-59024)
Patent Document 5: Japanese Patent No. 4088036
Patent Document 6: International Publication No. WO2010/126170
Patent Document 7: Japanese Patent Application Laid-Open No. 2010-260805 (JP 2010-260805 A)

### Non-Patent Document

Non-Patent Document 1: Tetrahedron Lett., No. 42, 1970, pp. 3719-3722
Non-Patent Document 2: Chem. Commun., 2002, pp. 1872-1873

### Summary of Invention

### Technical Problem

An object of the present invention is to provide a safer industrial process for producing an isothiazole compound, particularly 3,4-dichloro-5-cyanoisothiazole, by avoiding the simultaneous use of an aprotic polar solvent such as N,N-dimethylformamide and chlorine.

Another object of the present invention is to provide a process for producing an isothiazole compound, particularly 3,4-dichloro-5-cyanoisothiazole, which is economically preferred because an aprotic polar solvent such as N,N-dimethylformamide, which is highly likely to become a part of waste, is not used.

Still another object of the present invention is to provide a process for producing an isothiazole compound, particularly 3,4-dichloro-5-cyanoisothiazole, which process does not substantially use a raw material, which may require attention and measures, or a special raw material. An example of the raw material which may require attention and measures is an inorganic cyanide having extremely high toxicity, which is a source for hydrocyanic acid gas and cyanide ions. In addition, another example of the raw material which may require attention and measures is a special inflammable material. In addition, still another example of the raw material which may require attention and measures is an organic compound having an industrially significant sublimation property.

Still another object of the present invention is to provide a process for producing an isothiazole compound, particularly 3,4-dichloro-5-cyanoisothiazole, which process is suitable for industrialization because of a simple operation.

For example, the production process described in the above Patent Document 6 requires two steps from succinonitrile in order to produce 3,4-dichloro-5-cyanoisothiazole. However, to provide a process which can produce, for example, 3,4-dichloro-5-cyanoisothiazole, in a simple manner in only one step from succinonitrile is one of the objects of the present invention.

In short, an object of the present invention is to provide an industrially preferred process for producing an isothiazole compound, particularly 3,4-dichloro-5-cyanoisothiazole.

### Solution to Problem

In view of the circumstances as described above, the present inventor has earnestly studied processes for producing an isothiazole compound represented by a general formula (3) described later. As a result, the present inventor has unexpectedly found that the isothiazole compound represented by the general formula (3) described later can be produced by introducing a halogen represented by a general formula (2) described later into a nitrile compound represented by a general formula (1) described later and sulfur at 70°C or more, in the presence of an ether compound represented by a general formula (7) described later, to carry out a reaction between the nitrile compound, the sulfur and the halogen. Particularly, the present inventor has unexpectedly found that 3,4-dichloro-5-cyanoisothiazole represented by a formula (6) described later can be produced by introducing chlorine represented by a formula (5) described later into succinonitrile represented by a formula (4) described later and sulfur at 70°C or more, in the presence of an ether compound represented by a general formula (7) described later, to carry out a reaction between the succinonitrile, the sulfur and the chlorine. Based on these findings, the present inventor has completed the present invention. That is, the present invention is as follows:
[1] A process for producing an isothiazole compound represented by a general formula (3): 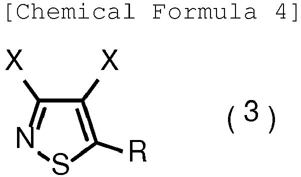 (wherein R is a cyano group, a carboxy group or an alkoxycarbonyl group; and X is a fluorine atom, a chlorine atom, a bromine atom or an iodine atom)
   which comprises introducing a halogen represented by a general formula (2):
   [Chemical Formula 3]

   X₂ (2)

   (wherein X is as defined above)
   into a nitrile compound represented by a general formula (1): 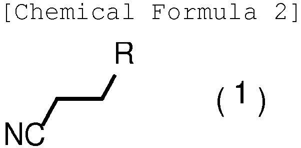 (wherein R is as defined above)
   and sulfur at 70°C or more in the presence of an ether compound represented by a general formula (7):
   [Chemical Formula 1]

   R¹-O-R² (7)

   (wherein R¹ and R² are the same or different and each represent an alkyl group or a cycloalkyl group)
   to carry out a reaction between the nitrile compound, the sulfur and the halogen.
[2] The process according to [1], wherein the introduction of the halogen into the nitrile compound and the sulfur in the presence of the ether compound is carried out at 90°C or more.
[3] The process according to [1], wherein the introduction of the halogen into the nitrile compound and the sulfur in the presence of the ether compound is carried out at a temperature in the range of 70 to 180°C.
[4] The process according to [1], wherein the introduction of the halogen into the nitrile compound and the sulfur in the presence of the ether compound is carried out at a temperature in the range of 90 to 150°C.
[5] The process according to any one of [1] to [4], wherein the introduction of the halogen into the nitrile compound and the sulfur in the presence of the ether compound is carried out for 15 hours or more.
[6] The process according to any one of [1] to [4], wherein the introduction of the halogen into the nitrile compound and the sulfur in the presence of the ether compound is carried out for a time period in the range of 15 to 75 hours.
[7] The process according to any one of [1] to [4], wherein the introduction of the halogen into the nitrile compound and the sulfur in the presence of the ether compound is carried out for a time period in the range of 15 to 50 hours.
[8] The process according to any one of [1] to [4], wherein the introduction of the halogen into the nitrile compound and the sulfur in the presence of the ether compound is carried out for a time period in the range of 15 to 30 hours.
[9] The process according to any one of [1] to [4], wherein the introduction of the halogen into the nitrile compound and the sulfur in the presence of the ether compound is carried out for 10 hours or more.
[10] The process according to any one of [1] to [4], wherein the introduction of the halogen into the nitrile compound and the sulfur in the presence of the ether compound is carried out for a time period in the range of 10 to 75 hours.
[11] The process according to any one of [1] to [4], wherein the introduction of the halogen into the nitrile compound and the sulfur in the presence of the ether compound is carried out for a time period in the range of 10 to 50 hours.
[12] The process according to any one of [1] to [4], wherein the introduction of the halogen into the nitrile compound and the sulfur in the presence of the ether compound is carried out for a time period in the range of 10 to 30 hours.
[13] The process according to any one of [1] to [4], wherein the introduction of the halogen into the nitrile compound and the sulfur in the presence of the ether compound is carried out for a time period in the range of 0.1 seconds to 100 hours.
[14] The process according to any one of [1] to [4], wherein the introduction of the halogen into the nitrile compound and the sulfur in the presence of the ether compound is carried out for a time period in the range of 1 second to 100 hours.
[15] The process according to any one of [1] to [4], wherein the introduction of the halogen into the nitrile compound and the sulfur in the presence of the ether compound is carried out for a time period in the range of 1 second to 50 hours.
[16] The process according to any one of [1] to [15], wherein the reaction between the nitrile compound, the sulfur and the halogen is carried out by charging the nitrile compound, the sulfur and the ether compound, and then introducing the halogen therein.
[17] The process according to any one of [1] to [16], wherein R is a cyano group, and X is a chlorine atom.
[18] The process according to any one of [1] to [17], wherein R¹ and R² are the same or different and each represent a C1 to C8 alkyl group or a C3 to C8 cycloalkyl group.
[19] The process according to any one of [1] to [17], wherein R¹ and R² are the same or different and each represent a C1 to C8 alkyl group.
[20] The process according to any one of [1] to [17], wherein R¹ and R² are the same or different and each represent a C2 to C8 alkyl group.
[21] The process according to any one of [1] to [17], wherein R¹ and R² are the same or different and each represent a C4 to C8 alkyl group.
[22] The process according to any one of [1] to [17], wherein R¹ and R² are the same or different and each represent a C4 alkyl group.
[23] The process according to any one of [1] to [17], wherein R¹ and R² are each a butyl group.
[24] The process according to any one of [1] to [23], wherein an amount of the ether compound used is 0.01 mol or more and 10 mol or less based on 1 mol of the nitrile compound.
[25] The process according to any one of [1] to [23], wherein an amount of the ether compound used is 0.05 mol or more and 2 mol or less based on 1 mol of the nitrile compound.
[26] The process according to any one of [1] to [23], wherein an amount of the ether compound used is 0.3 mol or more and 1 mol or less based on 1 mol of the nitrile compound.
[27] The process according to any one of [1] to [26], wherein an amount of sulfur used is 1 mol or more and 10 mol or less, as sulfur atoms, based on 1 mol of the nitrile compound.
[28] The process according to any one of [1] to [26], wherein an amount of sulfur used is 2 mol or more and 10 mol or less, as sulfur atoms, based on 1 mol of the nitrile compound.
[29] The process according to any one of [1] to [26], wherein an amount of sulfur used is 2 mol or more and 4 mol or less, as sulfur atoms, based on 1 mol of the nitrile compound.
[30] The process according to any one of [1] to [29], wherein an amount of the halogen used is 4 mol or more and 15 mol or less based on 1 mol of the nitrile compound.
[31] The process according to any one of [1] to [29], wherein an amount of the halogen used is 7 mol or more and 15 mol or less based on 1 mol of the nitrile compound.
[32] The process according to any one of [1] to [29], wherein an amount of the halogen used is 7 mol or more and 10 mol or less based on 1 mol of the nitrile compound.
[33] The process according to any one of [1] to [32], wherein the reaction between the nitrile compound, the sulfur and the halogen is carried out without using an aprotic polar solvent.
[34] The process according to any one of [1] to [32], wherein the reaction between the nitrile compound, the sulfur and the halogen is carried out without using any of N,N-dimethylformamide, N,N-diethylformamide, N,N-dimethylacetamide, N,N-diethylacetamide, N-methylpyrrolidone, tetramethylurea, 1,3-dimethyl-2-imidazolidinone or a mixture thereof.
[35] The process according to any one of [1] to [32], wherein the reaction between the nitrile compound, the sulfur and the halogen is carried out without using any of N,N-dimethylformamide, N-methylpyrrolidone or a mixture thereof.
[36] The process according to any one of [1] to [32], wherein the reaction between the nitrile compound, the sulfur and the halogen is carried out without using N,N-dimethylformamide.

### Advantageous Effects of Invention

The present invention provides a novel industrial process for producing an isothiazole compound, particularly 3,4-dichloro-5-cyanoisothiazole.

According to the present invention, a safer process for producing an isothiazole compound, particularly 3,4-dichloro-5-cyanoisothiazole, is provided by avoiding the simultaneous use of an aprotic polar solvent such as N,N-dimethylformamide and chlorine. That is, according to the present invention, a process which does not require special attention or special measures in order to maintain safety is provided by not using an aprotic polar solvent such as N,N-dimethylformamide as a solvent.

Avoiding the use of an aprotic polar solvent such as N,N-dimethylformamide means significantly improved safety in the process of the present invention, i.e., the advantage of the process of the present invention, compared with prior art. In other words, it substantially reduces the risks of dangerous decomposition and the like during industrial manufacture. Therefore, the process of the present invention is safely applicable to a pilot plant or production on a larger scale such as industrial manufacture.

In addition, according to the process of the present invention, the target isothiazole compound, particularly 3,4-dichloro-5-cyanoisothiazole, can be produced without using an aprotic polar solvent such as N,N-dimethylformamide as a reaction solvent. Therefore, the process of the present invention is economically preferred compared with prior art. Particularly, with respect to an aprotic polar solvent such as N,N-dimethylformamide, recycle is accompanied by difficulty because of working-up using water. Therefore, it is highly possible that the aprotic polar solvent becomes a part of waste. However, in the process of the present invention, the target compound can be produced without using an aprotic polar solvent. Therefore, the process of the present invention can reduce waste. That is, the process of the present invention can reduce the environmental load.

Furthermore, according to the process of the present invention, the isothiazole compound, particularly 3,4-dichloro-5-cyanoisothiazole, can be produced without substantially using a raw material which may require attention and measures or a special raw material. An example of the raw material which may require attention and measures is an inorganic cyanide having extremely high toxicity, which is a source for hydrocyanic acid gas and cyanide ions. In addition, another example of the raw material which may require attention and measures is a special inflammable material. In addition, still another example of the raw material which may require attention and measures is an organic compound having an industrially significant sublimation property.

Besides, according to the process of the present invention, the target isothiazole compound can be produced by a simple operation in only one step from the nitrile compound as a starting material, by simultaneously reacting the nitrile compound, sulfur and the halogen. Particularly, according to the process of the present invention, the target 3,4-dichloro-5-cyanoisothiazole can be produced by a simple operation in only one step from succinonitrile as a starting material, by simultaneously reacting succinonitrile, sulfur and chlorine.

For the raw materials in the process of the present invention, for example, all of succinonitrile among nitrile compounds, sulfur, and chlorine among halogens are raw materials which are widely used in the chemical industry, and are not only easily available but inexpensive.

Furthermore, according to the process of the present invention, the isothiazole compound, particularly 3,4-dichloro-5-cyanoisothiazole, can be produced under conditions which do not require a significantly high temperature or the like and are suitable for industrialization. Specifically, for example, the process of the present invention does not require a high temperature of 200°C or more.

Furthermore, the process of the present invention can be implemented in a simple manner and on an industrial scale without requiring a special reaction apparatus.

Therefore, the process of the present invention has high industrial use value.

### Description of Embodiment

The present invention will be described in detail below.

The present invention relates to a process for producing an isothiazole compound represented by a general formula (3): 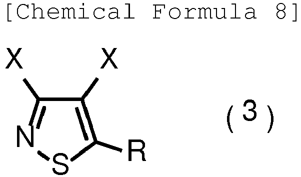 (wherein R is a cyano group, a carboxy group or an alkoxycarbonyl group; and X is a fluorine atom, a chlorine atom, a bromine atom or an iodine atom)
which comprises introducing a halogen represented by a general formula (2):
[Chemical Formula 7]

X₂ (2)

(wherein X is as defined above)
into a nitrile compound represented by a general formula (1): 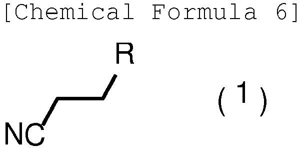 (wherein R is as defined above)
and sulfur at 70°C or more in the presence of an ether compound represented by a general formula (7):
[Chemical Formula 5]

R¹-O-R² (7)

(wherein R¹ and R² are the same or different and each represent an alkyl group or a cycloalkyl group)
to carry out a reaction between the nitrile compound, the sulfur and the halogen.

The process of the present invention particularly relates to a process for producing 3,4-dichloro-5-cyanoisothiazole represented by a formula (6): 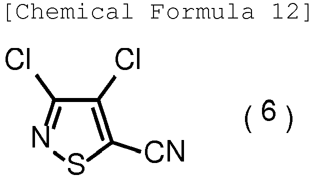 which comprises introducing chlorine represented by a formula (5) :
[Chemical Formula 11]

Cl₂ (5)

into succinonitrile represented by a formula (4): 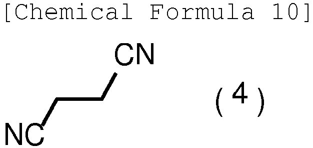 and sulfur at 70°C or more in the presence of an ether compound represented by a general formula (7):
[Chemical Formula 9]

R¹-O-R² (7)

(wherein R¹ and R² are the same or different and each represent an alkyl group or a cycloalkyl group)
to carry out a reaction between the succinonitrile, the sulfur and the chlorine.

The terms and symbols used in the present specification will be explained below.

"Ca to Cb" means that the number of carbon atoms "C" is "a" to "b". For example, "C1 to C4" of a "C1 to C4 alkyl" means that the number of carbon atoms in an alkyl is 1 to 4.

For a general term such as an "alkyl" in the present specification, for example, it is understood that "butyl" includes both straight chain and branched chain groups such as butyl and tert-butyl. However, when a specific term such as a "butyl group" is used, this is specific to a "normal butyl group", i.e., a "n-butyl group". In other words, the specific term "butyl group" means a "normal butyl group", a straight chain group, and when a branched chain isomer such as "tert-butyl" is intended, it is specifically mentioned.

The prefixes "n-", "s-" and "sec-", "i-", "t-" and "tert-", "neo-", and "c-" and "cyc-" have the following usual meanings of them: normal, secondary ("s-" and "sec-"), iso, tertiary ("t-" and "tert-"), neo and cyclo.

The "alkyl group" encompasses, but is not limited to, for example, a C1 to C8 alkyl group, a C2 to C8 alkyl group, a C4 to C8 alkyl group, a C4 alkyl group, a C1 to C4 alkyl group and the like.

The "C1 to C8 alkyl group" means a straight chain or branched chain alkyl group having 1 to 8 carbon atoms.

Specific examples of the "C1 to C8 alkyl group" encompass, but are not limited to, methyl, ethyl, propyl, isopropyl, butyl, sec-butyl, isobutyl, tert-butyl, pentyl, 1-methylbutyl, 2-methylbutyl, 3-methylbutyl, 1-ethylpropyl, 1,1-dimethylpropyl, 1,2-dimethylpropyl, neopentyl, hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1,2,2-trimethylpropyl, 1-ethyl-1-methylpropyl, 1-ethyl-2-methylpropyl, heptyl, 1-methylhexyl, 3-methylhexyl, 5-methylhexyl, 1,1-dimethylpentyl, 2,2-dimethylpentyl, 4,4-dimethylpentyl, 1-ethylpentyl, 2-ethylpentyl, 1,1,3-trimethylbutyl, 1,2,2-trimethylbutyl, 1,3,3-trimethylbutyl, 2,2,3-trimethylbutyl, 2,3,3-trimethylbutyl, 1-propylbutyl, 1,1,2,2-tetramethylpropyl, octyl, 1-methylheptyl, 3-methylheptyl, 6-methylheptyl, 2-ethylhexyl, 5,5-dimethylhexyl, 2,4,4-trimethylpentyl, 1-ethyl-1-methylpentyl, 1-propylpentyl and the like.

The "C2 to C8 alkyl group" means a straight chain or branched chain alkyl group having 2 to 8 carbon atoms.

Specific examples of the "C2 to C8 alkyl group" encompass, but are not limited to, alkyl groups having a corresponding number of carbon atoms among the specific examples of the C1 to C8 alkyl group described above.

The "C4 to C8 alkyl group" means a straight chain or branched chain alkyl group having 4 to 8 carbon atoms.

Specific examples of the "C4 to C8 alkyl group" encompass, but are not limited to, alkyl groups having a corresponding number of carbon atoms among the specific examples of the C1 to C8 alkyl group described above.

The "C4 alkyl group" means a straight chain or branched chain alkyl having 4 carbon atoms.

Specific examples of the "C4 alkyl group" encompass alkyl groups having a corresponding number of carbon atoms among the specific examples of the C1 to C8 alkyl group described above.

The "C1 to C4 alkyl group" means a straight chain or branched chain alkyl group having 1 to 4 carbon atoms.

Specific examples of the "C1 to C4 alkyl group" encompass alkyl groups having a corresponding number of carbon atoms among the specific examples of the C1 to C8 alkyl group described above.

The "cycloalkyl group" encompasses, but is not limited to, for example, a C3 to C8 cycloalkyl group, preferably a C5 to C6 cycloalkyl group and the like.

The "C3 to C8 cycloalkyl group" means a cycloalkyl group having 3 to 8 carbon atoms.

Specific examples of the "C3 to C8 cycloalkyl group" encompass cyclopropyl, cyclobutyl, cyclopentyl, cyclohexyl, cycloheptyl and cyclooctyl.

The "C5 to C6 cycloalkyl group" means cyclopentyl and cyclohexyl.

The "alkoxycarbonyl group" encompasses, for example, a C1 to C4 alkoxycarbonyl group and the like.

The "C1 to C4 alkoxycarbonyl group" means a (C1 to C4 alkyl) -O-C(=O)-group, in which the C1 to C4 alkyl group has the same meaning as described above.

Specific examples of the "C1 to C4 alkoxycarbonyl group" encompass methoxycarbonyl, ethoxycarbonyl, propoxycarbonyl, isopropoxycarbonyl, butoxycarbonyl, sec-butoxycarbonyl, isobutoxycarbonyl and tert-butoxycarbonyl, and preferably, methoxycarbonyl, ethoxycarbonyl, propoxycarbonyl and isopropoxycarbonyl.

The "aprotic polar solvent" encompasses, for example, an amide-based aprotic polar solvent (e.g., N,N-dimethylformamide (DMF), N,N-diethylformamide, N,N-dimethylacetamide (DMAC), N,N-diethylacetamide, N-methylpyrrolidone (NMP), tetramethylurea, 1,3-dimethyl-2-imidazolidinone (DMI), hexamethylphosphoric triamide (HMPA), etc.), a sulfur-containing aprotic polar solvent (e.g., dimethyl sulfoxide (DMSO), sulfolane, dimethyl sulfone, etc.), a carbonate-based aprotic polar solvent (e.g., ethylene carbonate, propylene carbonate, etc.) and the like.

### (Raw Material Compounds)

The raw materials in the process of the present invention will be described.

### (Nitrile Compound)

As a raw material in the process of the present invention, the nitrile compound represented by the above general formula (1) is used. In the formula (1), R is a cyano group, a carboxy group or an alkoxycarbonyl group. Therefore, examples of the nitrile compound represented by the above general formula (1) include, but are not limited to, succinonitrile, 3-cyanopropionic acid, methyl 3-cyanopropionate, ethyl 3-cyanopropionate, propyl 3-cyanopropionate, isopropyl 3-cyanopropionate and butyl 3-cyanopropionate.

From the viewpoints of availability, price, the usefulness of the product, etc., as the nitrile compound used in the process of the present invention, the succinonitrile represented by the above formula (4) is particularly preferred. The succinonitrile is currently industrially available at a relatively low cost. Furthermore, the succinonitrile is preferred as an industrial raw material also from the aspects of the handling and toxicity thereof.

### (Sulfur)

The sulfur used in the process of the present invention will be described. Elemental sulfur is used in the process of the present invention. The form of the sulfur used in the process of the present invention is not particularly limited, and may be any form as long as the reaction proceeds.

The amount of sulfur used in the process of the present invention may be any amount as long as the reaction proceeds. From the viewpoints of yield and/or the suppression of by-products, economic efficiency, etc., as the amount of sulfur used in the process of the present invention, 0.9 mol or more, preferably 1 mol or more, and more preferably 2 mol or more, as sulfur atoms, based on 1 mol of the nitrile compound represented by the general formula (1), can be mentioned as examples.

From the viewpoints of yield and/or the suppression of by-products, economic efficiency, etc., as the amount of sulfur used in the process of the present invention, 20 mol or less, preferably 10 mol or less, and more preferably 4 mol or less, as sulfur atoms, based on 1 mol of the nitrile compound represented by the general formula (1), can be mentioned as examples.

Therefore, as the range of the amount of sulfur used in the process of the present invention, any appropriate combination of a lower limit and an upper limit which are described above can be mentioned as examples. The range of 0.9 mol or more and 20 mol or less, preferably 1 mol or more and 10 mol or less, more preferably 2 mol or more and 10 mol or less, and further preferably 2 mol or more and 4 mol or less, as sulfur atoms, based on 1 mol of the nitrile compound represented by the general formula (1), can be mentioned as examples. However, the amount of sulfur used in the process of the present invention can be adjusted appropriately by a person skilled in the art in accordance with the purpose and the situation.

### (Halogen)

The halogen used in the process of the present invention will be described. The halogen represented by the above general formula (2) is used in the process of the present invention.

As examples of the halogen which can be used in the process of the present invention, fluorine, chlorine, bromine and iodine are mentioned.

From the viewpoints of availability, ease of handling, price, the usefulness of the product, etc., as the halogen used in the process of the present invention, the chlorine represented by the above formula (5) or bromine is preferred, and the chlorine represented by the above formula (5) is particularly preferred.

The form of the halogen used in the process of the present invention is not particularly limited, and may be any form as long as the reaction proceeds. Examples of the form of the halogen used in the process of the present invention include a gas, a liquid and a solid.

Particularly, also the form of the chlorine used in the process of the present invention is not particularly limited, and may be any form as long as the reaction proceeds. Examples of the form of the chlorine used in the process of the present invention include a liquid and a gas. Preferred examples of the form of the chlorine used in the process of the present invention include a gas. The method for introducing chlorine gas is not limited, and, for example, the method for introducing chlorine gas may be any of blowing into the gas phase of the reaction system, or blowing into the liquid phase of the reaction system (e.g., bubbling). Furthermore, when chlorine gas is blown into the liquid phase, an apparatus in which fine bubbles of chlorine gas are generated, or the like may be used. For example, when chlorine gas is blown into the liquid phase of the reaction system, the chlorine gas can be blown therein through a nozzle; the chlorine gas can be blown therein in the form of fine bubbles through a porous element provided at the end of a nozzle; a pipe having numerous holes can be provided in the reaction vessel so that the chlorine gas is blown out of the numerous small holes on the pipe forming bubbles of an appropriately small size; or other various apparatus measures can be taken. In addition, the chlorine gas may be diluted with a gas other than chlorine gas. Examples of the gas used for the dilution of the chlorine gas include, but are not limited to, inert gases such as nitrogen and argon. From the viewpoints of availability, ease of handling, safety, price, etc., nitrogen is preferred. The gas(es) used for the dilution of the chlorine gas may be used singly or as a mixture thereof in any ratio.

The amount of the halogen used in the process of the present invention may be any amount as long as the reaction proceeds. From the viewpoints of yield and/or the suppression of by-products, economic efficiency, etc., as the amount of the halogen represented by the general formula (2) used in the process of the present invention, the range of 1 mol or more and 60 mol or less, preferably 2 mol or more and 20 mol or less, more preferably 4 mol or more and 15 mol or less, further preferably 7 mol or more and 15 mol or less, and particularly preferably 7 mol or more and 10 mol or less, based on 1 mol of the nitrile compound represented by the general formula (1), can be mentioned as examples. However, the amount of the halogen used in the process of the present invention can be adjusted appropriately by a person skilled in the art in accordance with the purpose and the situation.

### (Operation: Introduction of Halogen)

In the process of the present invention, it is preferred that the nitrile compound, sulfur and the halogen are allowed to react simultaneously with each other in the presence of the ether compound. Particularly, in the process of the present invention, it is preferred that succinonitrile, sulfur, and chlorine are allowed to react simultaneously with each other in the presence of the ether compound. Therefore, in the process of the present invention, it is preferred to introduce the halogen into the nitrile compound and sulfur in the presence of the ether compound. More specifically, it is preferred to charge the nitrile compound, sulfur and the ether compound, and then introduce the halogen therein. In other words, it is preferred to introduce the halogen into the nitrile compound, sulfur and the ether compound. It is possible to charge the total amount of the nitrile compound, the total amount of sulfur and the total amount of the ether compound, and then introduce the total amount of the halogen. Or it is possible to charge a part of the nitrile compound, a part of sulfur and a part of the ether compound, and then introduce a part of the halogen, and then charge the remaining nitrile compound, the remaining sulfur and the remaining ether compound, and then introduce the remaining halogen. Furthermore, the charge of the nitrile compound, sulfur and the ether compound and the introduction of the halogen may be repeated. Furthermore, it is possible to charge the total amount of the ether compound, and then charge the nitrile compound and sulfur in portions, and introduce the halogen in portions. In all these cases, the respective amounts of the nitrile compound, sulfur and the ether compound charged at one time can be adjusted appropriately by a person skilled in the art. The amount of chlorine introduced at one time can also be adjusted appropriately by a person skilled in the art. As long as the reaction proceeds, the methods of these charge and introduction may be selected and adjusted appropriately by a person skilled in the art.

### (Ether Compound)

The ether compound used in the process of the present invention will be described. The ether compound represented by the above general formula (7) is used in the process of the present invention.

In the general formula (7), R¹ and R² are the same or different and each represent an alkyl group or a cycloalkyl group.

From the viewpoints of yield, ease of handling, economic efficiency, etc., R¹ and R² are preferably the same or different and each represent a C1 to C8 alkyl group or a C3 to C8 cycloalkyl group. R¹ and R² are more preferably the same or different and each represent a C1 to C8 alkyl group. R¹ and R² are further preferably the same or different and each represent a C2 to C8 alkyl group. R¹ and R² are further preferably the same or different and each represent a C4 to C8 alkyl group. R¹ and R² are further preferably the same or different and each represent a C4 alkyl group. R¹ and R² are particularly preferably each a butyl group.

The ether compound which can be used in the process of the present invention encompasses, but is not limited to, for example,
dipropyl ether and isomers thereof (e.g., diisopropyl ether etc.), dibutyl ether and isomers thereof (e.g., di-sec-butyl ether, diisobutyl ether, di-tert-butyl ether, butyl (sec-butyl) ether, butyl (isobutyl) ether, butyl (tert-butyl) ether, (sec-butyl) (isobutyl) ether, etc.),
dipentyl ether and isomers thereof (e.g., di(2-methylbutyl) ether, di(3-methylbutyl) ether, pentyl (2-methylbutyl) ether, (2-methylbutyl) (3-methylbutyl) ether, etc.),
dihexyl ether and isomers thereof,
diheptyl ether and isomers thereof,
dioctyl ether and isomers thereof,
methyl (octyl) ether and isomers thereof, ethyl (hexyl) ether and isomers thereof, ethyl (octyl) ether and isomers thereof, propyl (pentyl) ether and isomers thereof, propyl (hexyl) ether and isomers thereof,
dicyclopentyl ether, dicyclohexyl ether,
cyclopentyl (methyl) ether (CPME), cyclopentyl (ethyl) ether, cyclopentyl (propyl) ether and isomers thereof, butyl (cyclopentyl) ether and isomers thereof,
cyclohexyl (methyl) ether, cyclohexyl (ethyl) ether, cyclohexyl (propyl) ether and isomers thereof, butyl (cyclohexyl) ether and isomers thereof and the like.

From the viewpoints of yield, ease of handling, economic efficiency, etc., specific examples of a preferred ether compound used in the process of the present invention encompass dibutyl ether, di-sec-butyl ether, diisobutyl ether, di-tert-butyl ether, dipentyl ether, dihexyl ether, diheptyl ether, dioctyl ether and the like, more preferably, dibutyl ether, di-sec-butyl ether, diisobutyl ether and di-tert-butyl ether, and further preferably dibutyl ether.

The amount of the ether compound represented by the general formula (7) used in the process of the present invention may be any amount as long as the reaction proceeds. From the viewpoints of yield and/or the suppression of by-products, economic efficiency, etc., as the amount of the ether compound represented by the general formula (7) used in the process of the present invention, the following can be mentioned as examples. In one aspect, the ranges of 0.01 mol or more and 10 mol or less, and 0.01 mol or more and 5 mol or less, preferably, 0.05 mol or more and 5 mol or less, 0.05 mol or more and 2 mol or less, and 0.05 mol or more and 1 mol or less, and more preferably, 0.3 mol or more and 2 mol or less, and 0.3 mol or more and 1 mol or less, based on 1 mol of the nitrile compound represented by the general formula (1), can be mentioned as examples. In other words, the ranges of 1 mol% or more and 1000 mol% or less, and 1 mol% or more and 500 mol% or less, preferably, 5 mol% or more and 500 mol% or less, 5 mol% or more and 200 mol% or less, and 5 mol% or more and 100mol % or less, and more preferably, 30 mol% or more and 200 mol% or less, and 30 mol% or more and 100 mol% or less, based on the nitrile compound represented by the general formula (1), can be mentioned as examples. In another aspect, the ranges of 0.01 L or more and 5 L or less, 0.01 L or more and 1 L or less, 0.01 L or more and 0.5 L or less, and 0.01 L or more and 0.2 L or less, and preferably, 0.05 L or more and 2 L or less, 0.05 L or more and 1 L or less, 0.05 L or more and 0.5 L or less, and 0.05 L or more and 0.2 L or less, based on 1 mol of the nitrile compound represented by the general formula (1), can be mentioned as examples. In other words, the ranges of 0.01 L/mol or more and 5 L/mol or less, 0.01 L/mol or more and 1 L/mol or less, 0.01 L/mol or more and 0.5 L/mol or less, and 0.01 L/mol or more and 0.2 L/mol or less, and preferably, 0.05 L/mol or more and 2 L/mol or less, 0.05 L/mol or more and 1 L/mol or less, 0.05 L/mol or more and 0.5 L/mol or less, and 0.05 L/mol or more and 0.2 L/mol or less, based on the nitrile compound represented by the general formula (1), can be mentioned as examples. However, the amount of the ether compound used in the process of the present invention can be adjusted appropriately by a person skilled in the art in accordance with the purpose and the situation.

### (Temperature)

In the process of the present invention, the temperature of the introduction of the halogen into the nitrile compound and sulfur in the presence of the ether compound, and the temperature of the reaction between the nitrile compound, the sulfur and the halogen are not particularly limited as long as the reaction proceeds. From the viewpoints of yield and/or the suppression of by-products, operability, economic efficiency, etc., as the lower limit of each of the temperature of the introduction and the reaction temperature, 50°C or more, preferably 70°C or more, more preferably 80°C or more, and further preferably 90°C or more can be mentioned as examples. From the same viewpoints, as the upper limit of each of the temperature of the introduction and the reaction temperature, 200°C or less, preferably 180°C or less, more preferably 160°C or less, and further preferably 150°C or less can be mentioned as examples. As the range of each of the temperature of the introduction and the reaction temperature, a range determined by an arbitrary combination of a lower limit and an upper limit described above can be mentioned as examples. For example, a range determined by a combination of the preferred lower limit and the preferred upper limit described above is preferred, a range determined by a combination of the more preferred lower limit and the more preferred upper limit described above is more preferred, and a range determined by a combination of the further preferred lower limit and the further preferred upper limit described above is further preferred. Specifically, the range of 50 to 200°C, preferably 70 to 180°C, more preferably 80 to 160°C, and further preferably 90 to 150°C can be mentioned as examples, but the range of each of the temperature of the introduction and the reaction temperature is not limited to these. The temperature of the introduction of the halogen, particularly chlorine, and the reaction temperature in the process of the present invention are substantially the same. That is, the temperature of the introduction of the halogen, particularly chlorine, and the reaction temperature in the process of the present invention may be regarded as the same.

### (Time)

In the process of the present invention, the time of the introduction of the halogen into the nitrile compound and sulfur in the presence of the ether compound, and the time of the reaction between the nitrile compound, the sulfur and the halogen are not particularly limited as long as the reaction proceeds. From the viewpoints of yield and/or the suppression of by-products, economic efficiency, etc., particularly from the viewpoint of improvement in yield, as the lower limit of the time of the introduction, 5 hours or more, preferably 10 hours or more, more preferably 15 hours or more, and further preferably 20 hours or more can be mentioned as examples. In addition, the upper limit of the time of the introduction is not particularly limited, and also from the viewpoints of the suppression of the decomposition of the target compound, etc., and a general economic viewpoint, 100 hours or less, preferably 75 hours or less, more preferably 50 hours or less, and further preferably 30 hours or less can be mentioned as examples. As the range of the time of the introduction, any appropriate combination of a lower limit and an upper limit which are described above can be mentioned as examples. In one aspect, the range of 5 to 100 hours, preferably 10 to 100 hours, more preferably 10 to 75 hours, further preferably 10 to 50 hours, and particularly preferably 10 to 30 hours can be mentioned as examples, but the time of the introduction is not limited to these. In another aspect, the range of 5 to 100 hours, preferably 15 to 100 hours, more preferably 15 to 75 hours, further preferably 15 to 50 hours, and particularly preferably 15 to 30 hours can be mentioned as examples, but the time of the introduction is not limited to these. In still another aspect, the ranges of 0.1 seconds to 100 hours, 1 second to 100 hours, and 1 minute to 100 hours, and in addition, 0.1 seconds to 50 hours, 1 second to 50 hours, and 1 minute to 50 hours can be mentioned as examples, but the time of the introduction is not limited to these. The time of the introduction of the halogen, particularly chlorine, and the reaction time in the process of the present invention are substantially the same. That is, the time of the introduction of the halogen, particularly chlorine, and the reaction time in the process of the present invention may be regarded as the same.

### (Isothiazole Compound)

Specific examples of the isothiazole compound represented by the general formula (3) obtained by the process of the present invention include, but are not limited to,
3,4-difluoro-5-cyanoisothiazole,
3,4-dichloro-5-cyanoisothiazole,
3,4-dibromo-5-cyanoisothiazole,
3,4-diiodo-5-cyanoisothiazole,
3,4-difluoro-5-carboxyisothiazole,
3,4-dichloro-5-carboxyisothiazole,
3,4-dibromo-5-carboxyisothiazole,
3,4-diiodo-5-carboxyisothiazole,
3,4-difluoro-5-methoxycarbonylisothiazole,
3,4-dichloro-5-methoxycarbonylisothiazole,
3,4-dibromo-5-methoxycarbonylisothiazole,
3,4-diiodo-5-methoxycarbonylisothiazole,
3,4-dichloro-5-ethoxycarbonylisothiazole,
3,4-dibromo-5-ethoxycarbonylisothiazole,
3,4-dichloro-5-propoxycarbonylisothiazole,
3,4-dichloro-5-isopropoxycarbonylisothiazole and
3,4-dichloro-5-butoxycarbonylisothiazole.

From the viewpoints of the usefulness of the compound, etc., 3,4-dichloro-5-cyanoisothiazole and 3,4-dibromo-5-cyanoisothiazole are preferred, and 3,4-dichloro-5-cyanoisothiazole is particularly preferred.

### Examples

Next, the process of the present invention will be specifically described with reference to Examples; however, the present invention is not limited in any way by these Examples.

### Example 1

### Process Using Dibutyl Ether

### (Production of 3,4-dichloro-5-cyanoisothiazole)

In a 300 mL four-necked flask equipped with a stirrer, a reflux condenser and a thermometer, 50.0 g (0.62 mol) of succinonitrile, 60.1 g (1.9 mol) of sulfur and 40.5 g (0.31 mol; 50 mol% based on 1 mol of succinonitrile; 0.08 L/mol based on 1 mol of succinonitrile) of dibutyl ether were charged. The temperature was raised to 100°C under stirring. It was observed that the succinonitrile melted. It was observed that most of the sulfur remained as a solid. When the stirring was temporarily stopped for observation, a dibutyl ether layer, a succinonitrile layer and sulfur were observed in order from the upper layer. The stirring was resumed, and therein was blown 385.9 g (5.44 mol) of chlorine at 100 to 105°C for 20 hours. The reaction mixture was cooled to room temperature, and diluted with 100 mL of ethyl acetate. The insolubles were removed by filtration to obtain the product as a brown ethyl acetate solution. The obtained ethyl acetate solution was analyzed by an HPLC absolute calibration curve method. As a result, the yield of 3,4-dichloro-5-cyanoisothiazole was 83% with respect to the theoretical amount calculated from the amount of the succinonitrile used.

### Example 2

### Process Using Dibutyl Ether

### (Production of 3,4-dichloro-5-cyanoisothiazole)

In a 500 mL four-necked flask equipped with a stirrer, a reflux condenser and a thermometer, 80.1 g (1 mol) of succinonitrile, 99.2 g (3.1 mol) of sulfur and 19.0 mL (0.112 mol; 11.2 mol% based on 1 mol of succinonitrile; 0.019 L/mol based on 1 mol of succinonitrile) of dibutyl ether were charged. The temperature was raised to 120°C under stirring. It was observed that the succinonitrile melted. It was observed that the sulfur also melted. When the stirring was temporarily stopped for observation, a dibutyl ether layer, a succinonitrile layer and a sulfur layer were observed in order from the upper layer. The stirring was resumed, and therein was blown 390.0 g (5.50 mol) of chlorine at 120 to 125°C for 27 hours. The reaction mixture was cooled to room temperature, and diluted with 200 mL of toluene. The insolubles were removed by filtration to obtain the product as a brown toluene solution. The obtained toluene solution was analyzed by a GC absolute calibration curve method. As a result, the yield of 3,4-dichloro-5-cyanoisothiazole was 64% with respect to the theoretical amount calculated from the amount of the succinonitrile used.

When the production of an isothiazole compound was carried out according to the process of International Publication No. WO2010/126170 (Patent Document 6), the sublimation of a compound presumed to be fumaronitrile, maleonitrile and/or a chlorine-substituted compound thereof was observed. However, in Examples 1 and 2 of the present specification, the sublimation of a compound was not substantially observed.

The reaction mechanism and the like in the present invention were not clear, but from the above observation, it was presumed that the main reaction mechanism in the present invention was different from the main reaction mechanism in the process of International Publication No. WO2010/126170 (Patent Document 6).

### Comparative Example 1

### Process Described in Example 2 of Japanese Patent Application Laid-Open No. 2010-260805 (JP 2010-260805 A) (Patent Document 7)

### (Production of 3,4-dichloro-5-cyanoisothiazole)

In a 300 mL four-necked flask equipped with a stirrer, a reflux condenser and a thermometer, 5.70 g (71.0 mmol) of succinonitrile, 35.5 mL (0.5 L/mol based on 1 mol of succinonitrile) of N,N-dimethylformamide and 36.5 g (1.14 mol) of sulfur were charged. Therein was blown 40.4 g (0.570 mol) of chlorine at 25°C or less under stirring. Then, the temperature was raised to 100°C, and the mixture was stirred for 6 hours. The reaction mixture was allowed to cool to 25°C, and then poured into ice water. The reaction product was extracted with toluene. The obtained toluene solution was analyzed by an HPLC absolute calibration curve method. As a result, the yield of 3,4-dichloro-5-cyanoisothiazole was 64% with respect to the theoretical amount calculated from the amount of the succinonitrile used.

In the above Comparative Example 1 which is the process described in Example 2 of Japanese Patent Application Laid-Open No. 2010-260805 (JP 2010-260805 A) (Patent Document 7), chlorine and N,N-dimethylformamide are used. Therefore, as described earlier, the process of Comparative Example 1 is different from the present invention which does not require the use of N,N-dimethylformamide, and is not industrially preferred.

It was presumed that in the above Comparative Example 1, sulfur chloride was produced from the chlorine and the sulfur in the system at a low temperature of 25°C or less, and then the succinonitrile reacted with the sulfur chloride prepared in the system, as described in claims 1 and 6 and the paragraph 0029 of Japanese Patent Application Laid-Open No. 2010-260805 (JP 2010-260805 A) (Patent Document 7).

Meanwhile, as described already, the reaction mechanism and the like in the present invention are not clear. However, when the present invention was discussed after the present invention was completed, it was presumed that the main reaction mechanism in the present invention was different from the main reaction mechanism in the above Comparative Example 1. This is supported also by Comparative Example 2 described later.

### Comparative Example 2

### Process Described in Example 2 of Japanese Patent Application Laid-Open No. 2010-260805 (JP 2010-260805 A) (Patent Document 7) Conducted Using Dibutyl Ether Instead of N,N-Dimethylformamide

### (Production of 3,4-dichloro-5-cyanoisothiazole)

In a 50 mL eggplant-shaped flask equipped with a stirrer, a reflux condenser and a thermometer, 5.0 g (62.4 mmol) of succinonitrile, 31.9 g (1.0 mol) of sulfur and 31.0 mL (183 mmol; 0.5 L/mol based on 1 mol of succinonitrile) of dibutyl ether were charged. It was observed that most of the succinonitrile did not melt. It was observed that the sulfur did not melt. Therein was blown 35.4 g (0.50 mol) of chlorine at 25°C or less under stirring for 22 hours. Then, the temperature was raised to 100°C, and the mixture was stirred for 6 hours. The reaction mixture was cooled to room temperature, and diluted with 50 mL of toluene. The insolubles were removed by filtration to obtain the product as a brown toluene solution. The obtained toluene solution was analyzed by a GC absolute calibration curve method. As a result, the yield of 3,4-dichloro-5-cyanoisothiazole was only 5.3% with respect to the theoretical amount calculated from the amount of the succinonitrile used.

In the above Comparative Example 2, the process described in Example 2 of Japanese Patent Application Laid-Open No. 2010-260805 (JP 2010-260805 A) (Patent Document 7) was conducted using dibutyl ether instead of N,N-dimethylformamide, which is an aprotic polar solvent. As a result, the yield decreased significantly.

### Comparative Example 3

### Process Using Diphenyl Ether

### (Production of 3,4-dichloro-5-cyanoisothiazole)

In a 500 mL four-necked flask equipped with a stirrer, a reflux condenser and a thermometer, 80.1 g (1 mol) of succinonitrile, 96.0 g (3.1 mol) of sulfur and 80 mL (0.51 mol; 51 mol% based on 1 mol of succinonitrile; 0.08 L/mol based on 1 mol of succinonitrile) of diphenyl ether were charged. The temperature was raised to 120°C under stirring. When the stirring was temporarily stopped for observation, the succinonitrile and the diphenyl ether had melted and dissolved in each other to form one layer. It was observed that the sulfur also melted. The mixture formed two layers. The upper layer was a layer of succinonitrile and diphenyl ether. The lower layer was a sulfur layer. Therein was blown 628.2 g (8.8 mol) of chlorine at 120 to 125°C under stirring for 44 hours. The reaction mixture was cooled to room temperature, and diluted with 200 mL of toluene. The insolubles were removed by filtration to obtain the product as a brown toluene solution. The obtained toluene solution was analyzed by a GC absolute calibration curve method. As a result, the yield of 3,4-dichloro-5-cyanoisothiazole was 39.0% with respect to the theoretical amount calculated from the amount of the succinonitrile used.

### Comparative Example 4

### Process Using Butanol

### (Production of 3,4-dichloro-5-cyanoisothiazole)

In a 300 mL four-necked flask equipped with a stirrer, a reflux condenser and a thermometer, 40.0 g (0.5 mol) of succinonitrile, 48.0 g (1.5 mol) of sulfur and 7.4 g (0.1 mol; 20 mol% based on 1 mol of succinonitrile; 0.018 L/mol based on 1 mol of succinonitrile) of butanol were charged. The temperature was raised to 100°C under stirring. When the stirring was temporarily stopped for observation, the succinonitrile and the butanol had dissolved in each other. It was confirmed that the sulfur remained as a solid. The stirring was resumed, and therein was blown 145.3 g (2.05 mol) of chlorine at 100 to 105°C for 18 hours. The reaction mixture was cooled to room temperature, and diluted with 100 mL of ethyl acetate. The insolubles were removed by filtration to obtain the product as a brown ethyl acetate solution. The obtained ethyl acetate solution was analyzed by an HPLC absolute calibration curve method. As a result, the yield of 3,4-dichloro-5-cyanoisothiazole was 54% with respect to the theoretical amount calculated from the amount of the succinonitrile used.

### Comparative Example 5

### Process Using Diglyme

### (Production of 3,4-dichloro-5-cyanoisothiazole)

In a 300 mL four-necked flask equipped with a stirrer, a reflux condenser and a thermometer, 80.1 g (1.0 mol) of succinonitrile, 32.1 g (1.0 mol) of sulfur and 134.17 g (1.0 mol; 100 mol% based on 1 mol of succinonitrile; 0.14 L/mol based on 1 mol of succinonitrile) of diglyme were charged. The temperature was raised to 120°C under stirring. It was observed that the succinonitrile melted. It was observed that the sulfur also melted. When the stirring was temporarily stopped for observation, a diglyme layer, a succinonitrile layer and a sulfur layer were observed in order from the upper layer. The stirring was resumed, and therein was blown 248.2 g (3.5 mol) of chlorine at 120 to 125°C for 24 hours. The reaction mixture was cooled to room temperature, and diluted with 300 mL of ethyl acetate. The insolubles were removed by filtration to obtain the product as a brown ethyl acetate solution. The obtained ethyl acetate solution was analyzed by an HPLC absolute calibration curve method. As a result, the yield of 3,4-dichloro-5-cyanoisothiazole was 46% with respect to the theoretical amount calculated from the amount of the succinonitrile used.

### (High-Performance Liquid Chromatography (HPLC) Analysis Method)

Regarding the details of the above-described HPLC analysis method, the following literatures can be referred to, if necessary.
(a): The Chemical Society of Japan ed., "Shin Jikken kagaku Koza (New Experimental Chemistry Course) 9 Bunseki kagaku (Analytical Chemistry) II", pages 86 to 112 (1977), published by Shingo Iizumi, Maruzen Co., Ltd. (For example, regarding combinations of packing materials and mobile phases that can be used in a column, pages 93 to 96 can be referred to.)
(b) : The Chemical Society of Japan ed., "Jikken kagaku Koza (Experimental Chemistry Course) 20-1 Bunseki kagaku (Analytical Chemistry)", 5th ed., pages 130 to 151 (2007), published by Seishiro Murata, Maruzen Co., Ltd. (For example, regarding the specific usage and conditions of reversed phase chromatography analysis, pages 135 to 137 can be referred to.)

### (Gas Chromatography (GC) Analysis Method)

Regarding the details of the above-described GC analysis method, the following literatures can be referred to, if necessary.
(a): The Chemical Society of Japan ed., "Shin Jikken kagaku Koza (New Experimental Chemistry Course) 9 Bunseki kagaku (Analytical Chemistry) II", pages 60 to 86 (1977), published by Shingo Iizumi, Maruzen Co., Ltd. (For example, regarding liquids for a stationary phase that can be used in a column, page 66 can be referred to.)
(b) : The Chemical Society of Japan ed., "Jikken kagaku Koza (Experimental Chemistry Course) 20-1 Bunseki kagaku (Analytical Chemistry)", 5th ed., pages 121 to 129 (2007), published by Seishiro Murata, Maruzen Co., Ltd. (For example, regarding the specific usage of a hollow capillary separation column, pages 124 to 125 can be referred to.)

### Industrial Applicability

According to the process of the present invention, a novel industrial process for producing an isothiazole compound, particularly 3,4-dichloro-5-cyanoisothiazole, is provided. The isothiazole compound which can be produced by the process of the present invention is useful as an intermediate for pharmaceuticals and an intermediate for agricultural chemicals, and an intermediate for functional pigments, electronic materials, etc. Particularly, 3,4-dichloro-5-cyanoisothiazole is useful as an important intermediate for agricultural chemicals.

As described earlier in this specification, the process of the present invention is industrially preferred. For example, the process of the present invention is dramatically safer compared with prior art, and is efficient. Therefore, the process of the present invention can be implemented on an industrial scale and in a simple manner. Furthermore, the process of the present invention is economically preferred. For example, an aprotic polar solvent such as N,N-dimethylformamide, which is highly likely to become a part of waste, is not used, and therefore the process of the present invention is economically preferred.

Besides, according to the process of the present invention, the target compound can be produced without using an expensive catalyst and a transition metal, and therefore, harmful waste derived from them is not discharged. Therefore, in the process of the present invention, waste disposal is easy, and the process of the present invention is also environmentally friendly.

Therefore, the process of the present invention is extremely useful as an industrial production process.

## Claims

1. A process for producing an isothiazole compound represented by a general formula (3): 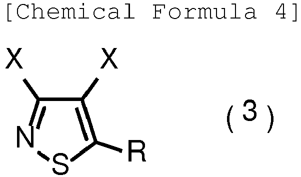 (wherein R is a cyano group, a carboxy group or an alkoxycarbonyl group; and X is a fluorine atom, a chlorine atom, a bromine atom or an iodine atom)
which comprises introducing a halogen represented by a general formula (2):
[Chemical Formula 3]
X₂ (2)
(wherein X is as defined above)
into a nitrile compound represented by a general formula (1): 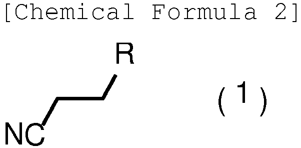 (wherein R is as defined above)
and sulfur at 70°C or more in the presence of an ether compound represented by a general formula (7):
[Chemical Formula 1]
R¹-O-R² (7)
(wherein R¹ and R² are the same or different and each represent an alkyl group or a cycloalkyl group)
to carry out a reaction between the nitrile compound, the sulfur and the halogen.

2. The process according to claim 1, wherein the introduction of the halogen into the nitrile compound and the sulfur in the presence of the ether compound is carried out at 90°C or more.

3. The process according to claim 1, wherein the introduction of the halogen into the nitrile compound and the sulfur in the presence of the ether compound is carried out at a temperature in the range of 70 to 180°C.

4. The process according to claim 1, wherein the introduction of the halogen into the nitrile compound and the sulfur in the presence of the ether compound is carried out at a temperature in the range of 90 to 150°C.

5. The process according to any one of claims 1 to 4, wherein the introduction of the halogen into the nitrile compound and the sulfur in the presence of the ether compound is carried out for 15 hours or more.

6. The process according to any one of claims 1 to 4, wherein the introduction of the halogen into the nitrile compound and the sulfur in the presence of the ether compound is carried out for a time period in the range of 15 to 75 hours.

7. The process according to any one of claims 1 to 4, wherein the introduction of the halogen into the nitrile compound and the sulfur in the presence of the ether compound is carried out for a time period in the range of 15 to 50 hours.

8. The process according to any one of claims 1 to 7, wherein R is a cyano group, and X is a chlorine atom.

9. The process according to any one of claims 1 to 8, wherein R¹ and R² are the same or different and each represent a C1 to C8 alkyl group or a C3 to C8 cycloalkyl group.

10. The process according to any one of claims 1 to 8, wherein R¹ and R² are the same or different and each represent a C1 to C8 alkyl group.

11. The process according to any one of claims 1 to 8, wherein R¹ and R² are the same or different and each represent a C4 to C8 alkyl group.

12. The process according to any one of claims 1 to 8, wherein R¹ and R² are each a butyl group.

## Patentansprüche

1. Verfahren zur Herstellung einer Isothiazolverbindung, die durch eine allgemeine Formel (3) dargestellt wird: 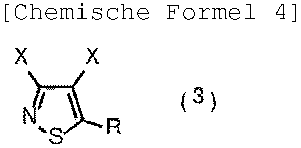 (wobei R eine Cyano-Gruppe, eine Carboxygruppe oder eine Alkoxycarbonyl-Gruppe ist; und X ein Fluoratom, ein Chloratom, ein Bromatom, oder ein Iodatom ist)
das Verfahren umfassend das Einbringen eines Halogens, dargestellt durch eine allgemeine Formel (2):
[Chemische Formel 3]
**X₂** **(2)**
(wobei x wie oben definiert ist)
in eine Nitrilverbindung, dargestellt durch eine allgemeine Formel (1): 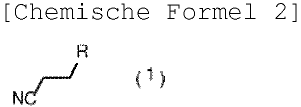 (wobei R wie oben definiert ist)
und Schwefel bei 70 °C oder mehr in der Gegenwart einer Etherverbindung, dargestellt durch eine allgemeine Formel (7)
[Chemische Formel 1]
R¹-O-R² (7)
(wobei R¹ und R² gleich oder verschieden sind und jeweils eine Alkylgruppe oder eine Cycloalkylgruppe darstellen) um eine Reaktion zwischen der Nitrilverbindung, dem Schwefel und dem Halogen durchzuführen.

2. Verfahren nach Anspruch 1, wobei die Einbringung des Halogens in die Nitrilverbindung und den Schwefel in Gegenwart der Etherverbindung bei 90 °C oder mehr erfolgt.

3. Verfahren nach Anspruch 1, wobei die Einbringung des Halogens in die Nitrilverbindung und den Schwefel in der Gegenwart der Etherverbindung bei einer Temperatur im Bereich von 70 bis 180 °C erfolgt.

4. Verfahren nach Anspruch 1, wobei die Einbringung des Halogens in die Nitrilverbindung und den Schwefel in der Gegenwart der Etherverbindung bei einer Temperatur im Bereich von 90 bis 150 °C erfolgt.

5. Verfahren nach einem der Ansprüche 1 bis 4, wobei die Einbringung des Halogens in die Nitrilverbindung und den Schwefel in der Gegenwart der Etherverbindung für 15 Stunden oder mehr erfolgt.

6. Verfahren nach einem der Ansprüche 1 bis 4, wobei die Einbringung des Halogens in die Nitrilverbindung und den Schwefel in der Gegenwart der Etherverbindung für einen Zeitraum im Bereich von 15 bis 75 Stunden erfolgt.

7. Verfahren nach einem der Ansprüche 1 bis 4, wobei die Einbringung des Halogens in die Nitrilverbindung und den Schwefel in der Gegenwart der Etherverbindung für einen Zeitraum im Bereich von 15 bis 50 Stunden erfolgt.

8. Verfahren nach einem der Ansprüche 1 bis 7, wobei R eine Cyanogruppe ist, und X ein Chloratom ist.

9. Verfahren nach einem der Ansprüche 1 bis 8, wobei R¹ und R² gleich oder verschieden sind und jeweils eine C1-C8-Alkylgruppe, oder eine C3-C8-Cycloalkylgruppe darstellen.

10. Verfahren nach einem der Ansprüche 1 bis 8, wobei R¹ und R² gleich oder verschieden sind und jeweils eine C1-C8-Alkylgruppe darstellen.

11. Verfahren nach einem der Ansprüche 1 bis 8, wobei R¹ und R² gleich oder verschieden sind und jeweils eine C4-C8-Alkylgruppe darstellen.

12. Verfahren nach einem der Ansprüche 1 bis 8, wobei R¹ und R² jeweils eine Butylgruppe sind.

## Revendications

1. Procédé destiné à produire un composé isothiazole représenté par une formule générale (3) : 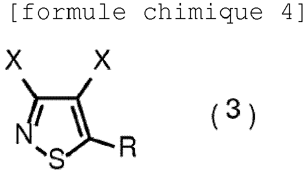 (dans lequel R est un groupe cyano, un groupe carboxy, ou un groupe alkoxycarbonyle, et X est un atome de fluor, un atome de chlore, un atome de brome, ou un atome d'iode), lequel comprend l'introduction d'un halogène représenté par une formule générale (2) :
[formule chimique 3]
X₂ (2)
(dans lequel X est comme défini plus haut)
dans un composé nitrile représenté par une formule générale (1) : 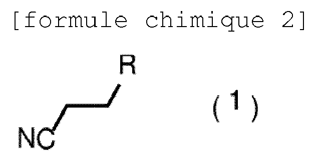 (dans lequel R est comme défini plus haut)
et du soufre à 70 °C ou plus, en présence d'un composé éther représenté par une formule générale (7) :
[formule chimique 1]
R¹-O-R² (7)
(dans lequel R¹ et R² sont identiques ou différents, et chacun représente un groupe alkyle ou un groupe cycloalkyle)
afin de réaliser une réaction entre le composé nitrile, le soufre, et l'halogène.

2. Procédé selon la revendication 1, dans lequel
l'introduction de l'halogène dans le composé nitrile et le soufre en présence du composé éther est réalisée à 90°C ou plus.

3. Procédé selon la revendication 1, dans lequel
l'introduction de l'halogène dans le composé nitrile et le soufre en présence du composé éther est réalisée à une température comprise dans la plage de 70 à 180 °C.

4. Procédé selon la revendication 1, dans lequel
l'introduction de l'halogène dans le composé nitrile et le soufre en présence du composé éther est réalisée à une température comprise dans la plage de 90 à 150 °C.

5. Procédé selon l'une quelconque des revendications 1 à 4, dans lequel l'introduction de l'halogène dans le composé nitrile et le soufre en présence du composé éther est réalisée pendant 15 heures ou plus.

6. Procédé selon l'une quelconque des revendications 1 à 4, dans lequel l'introduction de l'halogène dans le composé nitrile et le soufre en présence du composé éther est réalisée sur une période de temps dans la plage comprise entre 15 et 75 heures.

7. Procédé selon l'une quelconque des revendications 1 à 4, dans lequel l'introduction de l'halogène dans le composé nitrile et le soufre en présence du composé éther est réalisée sur une période de temps dans la plage comprise entre 15 et 50 heures.

8. Procédé selon l'une quelconque des revendications 1 à 7, dans lequel R est un groupe cyano, et X est un atome de chlore.

9. Procédé selon l'une quelconque des revendications 1 à 8, dans lequel R¹ et R² sont identiques ou différents, et chacun représente un groupe alkyle en C1 à C8, ou un groupe cycloalkyle en C3 à C8.

10. Procédé selon l'une quelconque des revendications 1 à 8, dans lequel R¹ et R² sont identiques ou différents, et chacun représente un groupe alkyle en C1 à C8.

11. Procédé selon l'une quelconque des revendications 1 à 8, dans lequel R¹ et R² sont identiques ou différents, et chacun représente un groupe alkyle en C4 à C8.

12. Procédé selon l'une quelconque des revendications 1 à 8, dans lequel R¹ et R² sont chacun un groupe butyle.
